# FASCICULE DE BREVET EUROPEEN

(11) **EP 1 891 933 B1**
(45) Date de publication et mention de la délivrance du brevet: **05.05.2010**
(21) Numéro de dépôt: 07113082.7
(22) Date de dépôt: 25.07.2007
(51) Int. Cl.: A61K 8/81, A61Q 5/10, A61K 8/41

(54) **Composition tinctoriale comprenant un colorant d'oxydation et un polymère amphotère comprenant des motifs acrylamide, halogénure de dialkyldiallylammonium et acide carboxylique vinylique**
Färbezusammensetzung enthaltend ein Oxidationsfärbemittel und ein amphoterisches Polymer mit Acrylamideinheiten, Dialkyldiallylammonium und Vinylcarbonsäure
Tinctorial composition containing an oxidation dye and an amphoteric polymer comprising units of acrylamide, dialkyldiallylammonium halide and vinyl carboxylic acid

(30) Priorité: 10.08.2006 FR 0607247
(43) Date de publication de la demande: 27.02.2008
(73) Titulaire: L'Oréal, 75008 Paris (FR)
(72) Inventeur: Cottard, François, 92400 Courbevoie (FR); Deconinck, Gautier, 95210, Saint-Gratien (FR)
(74) Mandataire: Dossmann, Gérard

(56) Documents cités:
- EP-A1- 0 521 665
- EP-A1- 0 522 755
- WO-A-02/45674
- WO-A-94/06403
- US-A1- 2004 133 996

## Description

La présente invention a pour objet une composition pour la teinture d'oxydation des fibres kératiniques, et en particulier des fibres kératiniques humaines telles que les cheveux, comprenant, dans un milieu approprié pour la teinture, au moins un colorant d'oxydation et au moins un polymère amphotère particulier.

Il est connu de teindre les fibres kératiniques et en particulier les cheveux humains avec des compositions tinctoriales contenant des colorants d'oxydation, en particulier des précurseurs de colorants d'oxydation et des modificateurs de coloration.

Les précurseurs de colorants d'oxydation, appelés généralement bases d'oxydation, sont initialement des composés incolores ou faiblement colorés qui, associés à des produits oxydants, peuvent donner naissance par un processus de condensation oxydative à des composés colorés et colorants. Ce sont en général des composés tels que les ortho- ou para-phénylènediamines, les ortho- ou para-aminophénols et les bases hétérocycliques.

On sait également que l'on peut faire varier les nuances obtenues avec ces bases d'oxydation en les associant à des coupleurs ou modificateurs de coloration, ces derniers étant généralement choisis parmi les méta-diaminobenzènes, les méta-aminophénols, les méta-diphénols et certains composés hétérocycliques tels que des composés indoliques.

La variété des molécules mises en jeu au niveau des bases d'oxydation et des coupleurs permet l'obtention d'une riche palette de couleurs.

La coloration dite "permanente" obtenue grâce à ces colorants d'oxydation, appelée également coloration d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui est en général différemment sensibilisée (c'est-à-dire abîmée) entre sa pointe et sa racine.

La demande de brevet EP 1 048 290 décrit une composition pour la teinture d'oxydation des cheveux comprenant un précurseur de colorant d'oxydation, un coupleur et un polymère amphotère comprenant des unités répétitives (a) d'acide acrylique et (b) d'un monomère cationique choisi parmi le chlorure de méthacrylamidopropyl triméthyl ammonium, le chlorure de diméthyl diallyl ammonium et leurs mélanges, le rapport molaire entre les unités (a) et les unités (b) étant supérieur ou égal à 1 / 3. Cette composition permet d'améliorer le dépôt des colorants sur les cheveux et de rendre ainsi la teinture plus efficace.

Le brevet US 2005/0015895 décrit une composition pour la teinture d'oxydation des cheveux comprenant un colorant d'oxydation et un terpolymère amphotère quaternaire comprenant les unités répétitives suivantes : (a) le chlorure de méthacrylamidopropyl triméthyl ammonium ou le chlorure de diméthyl diallyl ammonium, (b) l'acide acrylique ou le méthacrylate de sodium, et (c) l'acrylamide, avec un rapport (a) / (b) supérieur ou égal à 4. Cette composition permet d'obtenir un meilleur effet de conditionnement des fibres capillaire.

Enfin, le brevet FR 2 817 467 décrit une composition pour la teinture d'oxydation des fibres kératiniques comprenant un colorant d'oxydation, un polymère associatif et un polymère à motifs issus d'un monomère de type (i) acrylamide, (ii) halogénure de dialkyldiallylammonium, et (iii) acide carboxylique vinylique. Cette composition permet d'obtenir une application optimale sur les fibres, en particulier elle ne coule pas, sans pour autant altérer les qualités de la coloration.

Toutefois, les compositions pour la teinture d'oxydation de l'art antérieur sont souvent difficiles à employer. En particulier, après un temps de pose plus ou moins long, la composition appliquée sur la chevelure doit être éliminée par rinçage, et cette étape de rinçage est souvent longue et délicate, le produit s'avérant difficile à éliminer.

Le but de la présente invention est d'obtenir de nouvelles compositions pour la teinture d'oxydation des fibres kératiniques qui ne présentent pas les inconvénients de l'art antérieur. Plus particulièrement, le but de la présente invention est d'obtenir des compositions de teinture plus faciles et plus rapides à éliminer, qui restent néanmoins faciles à appliquer (notamment qui ne coulent pas et restent bien localisées au point d'application) et ce sans pour autant altérer la puissance, la chromaticité et la sélectivité de la coloration.

Ce but est atteint avec la présente invention qui a pour objet une composition pour la teinture d'oxydation des fibres kératiniques, et en particulier des fibres kératiniques humaines telles que les cheveux, comprenant, dans un milieu approprié pour la teinture, au moins un colorant d'oxydation et au moins un polymère amphotère comprenant la répétition de :
(i) un ou plusieurs motifs issus d'un monomère de type acrylamide,
(ii) un ou plusieurs motifs issus d'un monomère de type halogénure de dialkyldiallylammonium, et
(iii) au moins 50 % en moles de un ou plusieurs motifs issus d'un monomère de type acide carboxylique vinylique.

La composition conforme à la présente invention s'élimine facilement et rapidement lors de l'étape de rinçage, tout en conservant de bonnes propriétés rhéologiques. En outre, elle permet d'améliorer les propriétés cosmétiques des cheveux, notamment en ce qui concerne le démêlage et le lissage.

Enfin, les propriétés tinctoriales de cette composition sont très satisfaisantes, tant en ce qui concerne la sélectivité que l'intensité de la coloration obtenue.

La présente invention a également pour objet un procédé de teinture des fibres kératiniques mettant en oeuvre la composition conforme à l'invention.

La présente invention a aussi pour objet un dispositif à plusieurs compartiments pour la mise en oeuvre du procédé conforme à l'invention.

La présente invention a aussi pour objet l'utilisation pour la teinture d'oxydation des fibers kératiniques de la composition conforme à l'invention.

La présente invention a enfin pour objet l'utilisation, dans une composition de teinture d'oxydation, d'un polymère amphotère tel que décrit dans la présente demande, pour obtenir une élimination facile et rapide de la composition lors de l'étape de rinçage.

A moins d'une indication différente, les bornes des gammes de valeurs qui sont données dans le cadre de la présente invention sont incluses dans ces gammes.

Selon un mode de réalisation particulier, les motifs issus d'un monomère de type acrylamide du polymère amphotère utile dans le cadre de l'invention sont des motifs de structure (I) suivante : 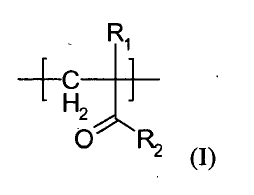 dans laquelle :
- R₁ désigne H ou CH₃,
- R₂ est choisi parmi un radical amino, diméthylamino, tert-butylamino, dodécylamino, ou -NH-CH₂OH.

De préférence, le polymère amphotère de l'invention ne comprend la répétition que d'un seul motif de formule (I).

Le motif issu d'un monomère de type acrylamide de formule (I) dans laquelle R₁ désigne H et R₂ est un radical amino est particulièrement préféré. Il correspond au monomère acrylamide proprement dit.

Selon un autre mode de réalisation particulier de l'invention, les motifs issus d'un monomère de type halogénure de dialkyldiallylammonium du polymère amphotère sont des motifs de structure (II) suivante : 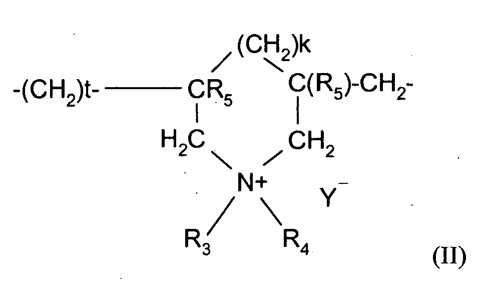 dans laquelle :
- k et t sont égaux à 0 ou 1, la somme k + t étant égale à 1 ;
- R₅ désigne un atome d'hydrogène ou un radical méthyle ;
- R₃ et R₄, indépendamment l'un de l'autre, désignent un groupement alkyle ayant de 1 à 4 atomes de carbone, un groupement hydroxyalkyle dans lequel le groupement alkyle a de préférence 1 à 5 atomes de carbone, un groupement amidoalkyle(C₁-C₄), ou R₃ et R₄ forment conjointement avec l'atome d'azote auquel ils sont rattachés, des groupements hétérocycliques, tels que pipéridinyle ou morpholinyle ; R₃ et R₄ indépendamment l'un de l'autre désignent de préférence un groupement alkyle ayant de 1 à 4 atomes de carbone;
- Y⁻ est un anion tel que bromure, chlorure, acétate, borate, citrate, tartrate, bisulfate, bisulfite, sulfate, phosphate.

Parmi ces motifs issus d'un monomère de type halogénure de dialkyldiallylammonium, on préfère ceux issus du monomère chlorure de diméthyldiallylammonium pour lesquels R₅ désigne un atome d'hydrogène et R₃ et R₄ désignent un radical méthyle, Y- désignant un anion chlorure.

Selon un mode de réalisation particulier de l'invention, les motifs issus d'un monomère de type acide carboxylique vinylique du polymère amphotère sont choisis parmi les motifs de formule (III) : 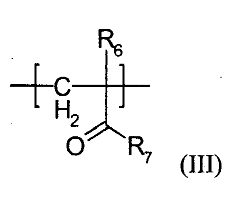 dans laquelle :
- R₆ désigne H ou CH₃,
- R₇ désigne un radical hydroxyle ou un radical -NH-C(CH₃)₂-CH₂-SO₃H.

Les motifs préférés correspondent aux monomères acide acrylique, acide méthacrylique et acide 2-acrylamido 2-méthyl propane sulfonique.

De préférence, le motif issu d'un monomère de type acide carboxylique vinylique est celui issu de l'acide acrylique pour lequel R₆ désigne un atome d'hydrogène et R₇ désigne un radical hydroxyle.

Selon l'invention, le ou les polymères amphotères comprennent au moins 50 % en moles de motifs issus d'un monomère de type acide carboxylique vinylique.

De préférence, ils comprennent de 50 à 90 % en moles de motifs issus d'un monomère de type acide carboxylique vinylique, de manière plus préférée de 50 à 75 % en moles.

Les teneurs des deux autres motifs peuvent avantageusement être les suivantes:
- de 1 à 40 %, préférentiellement de 5 à 25 % en moles de motifs issus d'un monomère de type acrylamide;
- de 1 à 40 %, préférentiellement de 5 à 25 % en moles de motifs issus d'un monomère de type halogénure de dialkyldiallylammonium.

Le ou les polymères amphotères utiles dans le cadre de l'invention peuvent également comprendre des motifs additionnels, différents des motifs issus d'un monomère de type acrylamide, halogénure de dialkyldiallylammonium, et acide carboxylique vinylique, du moment qu'il comprennent au moins 50 % en moles de motifs issus d'un monomère de type acide carboxylique vinylique.

Comme exemple de polymères à motifs issus d'un monomère de type (i) acrylamide, (ii) halogénure de dialkyldiallylammonium, et (iii) acide carboxylique vinylique, on peut citer notamment les terpolymères acrylamide / chlorure de diméthyldiallylammonium / acide acrylique, répertoriés dans le dictionnaire C.T.F.A. International Cosmetic Ingredient Dictionary, 10ème édition 2004, sous la dénomination "Polyquaternium 39". Les polymères selon l'invention peuvent ainsi être choisis parmi les Polyquaterniums 39 contenant au moins 50 % en moles de motifs issus de l'acide acrylique, tel que par exemple le produit commercialisé sous la dénomination MERQUAT 3333 par la société NALCO.

Le polymère amphotère selon l'invention peut être préparé de manière classique, par polymérisation à partir de ses différents monomères, selon des techniques connues de l'homme du métier et notamment par polymérisation radicalaire.

Le ou les polymères amphotères utiles dans le cadre de l'invention sont généralement présents en quantité comprise entre 0,1 et 10 % en poids, de préférence entre 0,5 et 5 % en poids, et plus particulièrement entre 1 et 4 % en poids par rapport au poids total de la composition de teinture.

La composition conforme à l'invention comprend au moins un colorant d'oxydation pouvant être choisi parmi les bases d'oxydation et les coupleurs.

Les bases d'oxydation utilisables dans le cadre de la présente invention sont choisies parmi celles classiquement connues en teinture d'oxydation, et parmi lesquelles on peut notamment citer les ortho- et para-phénylènediamines, les bases doubles, les ortho- et para- aminophénols, les bases hétérocycliques ainsi que les sels d'addition de ces composés avec un acide.

Les para-phénylènediamines utilisables dans le cadre de l'invention peuvent notamment être choisies parmi les composés de formule (IV) suivante et leurs sels d'addition avec un acide : 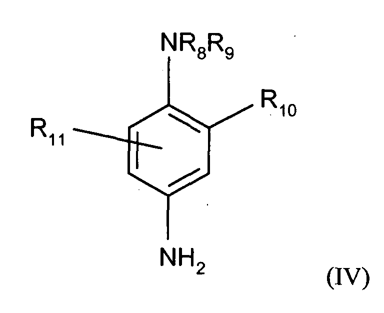 dans laquelle :
R₈ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄), alkyle en C₁-C₄ substitué par un groupement azoté, phényle ou 4'-aminophényle ;
R₉ représente un atome d'hydrogène, un radical alkyle en C₁-C₄, monohydroxyalkyle en C₁-C₄ ou polyhydroxyalkyle en C₂-C₄, alcoxy(C₁-C₄)alkyle(C₁-C₄) ou alkyle en C₁-C₄ substitué par un groupement azoté ;
R₈ et R₉ peuvent également former avec l'atome d'azote qui les porte un hétérocycle azoté à 5 ou 6 chaînons éventuellement substitué par un ou plusieurs groupements alkyle, hydroxy ou uréido ;
R₁₀ représente un atome d'hydrogène, un atome d'halogène tel qu'un atome de chlore, un radical alkyle en C₁-C₄, sulfo, carboxy, monohydroxyalkyle en C₁-C₄ ou hydroxyalcoxy en C₁-C₄, acétylaminoalcoxy en C₁-C₄, mésylaminoalcoxy en C₁-C₄ ou carbamoylaminoalcoxy en C₁-C₄;
R₁₁ représente un atome d'hydrogène, d'halogène ou un radical alkyle en C₁-C₄.

Parmi les groupements azotés de la formule (IV) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les paraphénylènediamines de formule (IV) ci-dessus, on peut plus particulièrement citer la paraphénylènediamine, la paratoluylènediamine, la 2-chloro-paraphénylènediamine, la 2,3-diméthyl-paraphénylènediamine, la 2,6-diméthyl-paraphénylènediamine, la 2,6-diéthyl-paraphénylènediamine, la 2,5-diméthyl-paraphénylènediamine, la N,N-diméthyl-paraphénylènediamine, la N,N-diéthyl-paraphénylènediamine, la N,N-dipropyl-paraphénylènediamine, la 4-amino-N,N-diéthyl-3-méthyl-aniline, la N,N-bis-(β-hydroxyéthyl)-paraphénylènediamine, la 4-N,N-bis-(β-hydroxyéthyl)amino-2-méthyl-aniline, la 4-N,N-bis-(β-hydroxyéthyl)-amino 2-chloro-aniline, la 2-β-hydroxyéthyl-paraphénylènediamine, la 2-fluoro-paraphénylènediamine, la 2-isopropyl-paraphénylènediamine, la N-(β-hydroxypropyl)-paraphénylènediamine, la 2-hydroxyméthyl-paraphénylènediamine, la N,N-diméthyl-3-méthyl-paraphénylènediamine, la N,N-(éthyl,β-hydroxyéthyl)-paraphénylènediamine, la N-(β,γ-dihydroxypropyl)-paraphénylènediamine, la N-(4'-aminophényl)-paraphénylènediamine, la N-phényl-paraphénylènediamine, la 2-β-hydroxyéthyloxy-paraphénylènediamine, la 2-β-acétylaminoéthyloxy-paraphénylènediamine, la N-(β-méthoxyéthyl)-paraphénylènediamine, la 2-méthyl-1-N-β-hydroxyéthyl-paraphénylènediamine, et leurs sels d'addition avec un acide.

Parmi les paraphénylènediamines de formule (IV) ci-dessus, on préfère tout particulièrement la paraphénylènediamine, la paratoluylènediamine, la 2-isopropyl-paraphénylènediamine, la 2-β-hydroxyéthyl-paraphénylènediamine, la 2-β-hydroxyéthyloxy-paraphénylènediamine, la 2,6-diméthyl-paraphénylène-diamine, la 2,6-diéthyl-paraphénylènediamine, la 2,3-diméthyl-paraphénylènediamine, la N,N-bis-(β-hydroxyéthyl)-paraphénylènediamine, la 2-chloro-paraphénylènediamine, et leurs sels d'addition avec un acide.

Selon l'invention, on entend par bases doubles les composés comportant au moins deux noyaux aromatiques sur lesquels sont portés des groupements amino et / ou hydroxyle.

Parmi les bases doubles utilisables à titre de bases d'oxydation dans la composition conforme à l'invention, on peut notamment citer les composés répondant à la formule (V) suivante et leurs sels d'addition avec un acide : 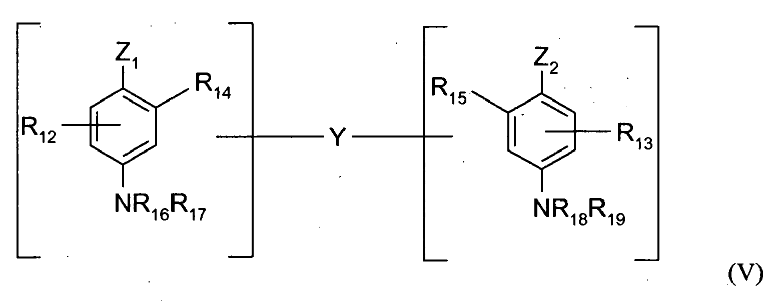 dans laquelle :
- Z₁ et Z₂, identiques ou différents, représentent un radical hydroxyle ou -NH₂ pouvant être substitué par un radical alkyle en C₁-C₄ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et / ou par un ou plusieurs hétéroatomes tels que des atomes d'oxygène, de soufre ou d'azote, et éventuellement substituée par un ou plusieurs radicaux hydroxyle ou alcoxy en C₁-C₆ ;
- R₁₂ et R₁₃ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
- R₁₄, R₁₅, R₁₆, R₁₇, R₁₈ et R₁₉, identiques ou différents, représentent un atome d'hydrogène, un bras de liaison Y ou un radical alkyle en C₁-C₄ ;
étant entendu que les composés de formule (V) ne comportent qu'un seul bras de liaison Y par molécule.

Parmi les groupements azotés de la formule (V) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les bases doubles de formules (V) ci-dessus, on peut plus particulièrement citer le N,N'-bis-(β-hydroxyéthyl)-N,N'-bis-(4'-aminophényl)-1,3-diamino-propanol, la N,N'-bis-(β-hydroxyéthyl)-N,N'-bis-(4'-aminophényl)-éthylènediamine, la N,N'-bis-(4-aminophényl)-tétraméthylènediamine, la N,N'-bis-(β-hydroxyéthyl)-N,N'-bis-(4-aminophényl)-tétraméthylènediamine, la N,N'-bis-(4-méthyl-aminophényl)-tétraméthylènediamine, la N,N'-bis-(éthyl)-N,N'-bis-(4'-amino-3'-méthylphényl)-éthylènediamine, le 1,8-bis-(2,5-diaminophénoxy)-3,5-dioxaoctane, et leurs sels d'addition avec un acide.

Parmi ces bases doubles de formule (V), le N,N'-bis-(β-hydroxyéthyl)-N,N'-bis-(4'-aminophényl)-1,3-diamino-propanol, le 1,8-bis-(2,5-diaminophénoxy)-3,5-dioxaoctane ou l'un de leurs sels d'addition avec un acide sont particulièrement préférés.

Les para-aminophénols utilisables dans le cadre de l'invention peuvent notamment être choisis parmi les composés répondant à la formule (VI) suivante et leurs sels d'addition avec un acide : 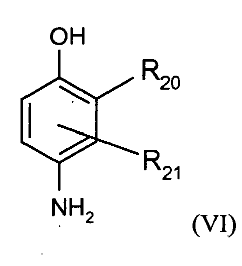 dans laquelle :
R₂₀ représente un atome d'hydrogène,un atome d'halogène tel que le fluor, un radical alkyle en C₁-C₄, monohydroxyalkyle en C₁-C₄, alcoxy(C₁-C₄)alkyle(C₁-C₄) ou aminoalkyle en C₁-C₄, ou hydroxyalkyl(C₁-C₄)aminoalkyle en C₁-C₄ ;
R₂₁ représente un atome d'hydrogène ou un atome d'halogène tel que le fluor, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄).

Parmi les para-aminophénols de formule (VI) ci-dessus, on peut plus particulièrement citer le para-aminophénol, le 4-amino-3-méthyl-phénol, le 4-amino-3-fluoro-phénol, le 4-amino-3-hydroxyméthyl-phénol, le 4-amino-2-méthyl-phénol, le 4-amino-2-hydroxyméthyl-phénol, le 4-amino-2-méthoxyméthyl-phénol, le 4-amino-2-aminométhyl-phénol, le 4-amino-2-(β-hydroxyéthyl-aminométhyl)-phénol, et leurs sels d'addition avec un acide.

Les ortho-aminophénols utilisables à titre de bases d'oxydation dans le cadre de la présente l'invention sont notamment choisis parmi le 2-amino-phénol, le 2-amino-1-hydroxy-5-méthyl-benzène, le 2-amino-1-hydroxy-6-méthyl-benzène, le 5-acétamido-2-amino-phénol, et leurs sels d'addition avec un acide.

Parmi les bases hétérocycliques utilisables à titre de bases d'oxydation dans la composition conforme à l'invention, on peut plus particulièrement citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diamino-pyridine, la 2-(4-méthoxyphényl)amino-3-amino-pyridine, la 2,3-diamino-6-méthoxy-pyridine, la 2-(β-méthoxyéthyl)amino-3-amino-6-méthoxy pyridine, la 3,4-diamino-pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-10659 ou demandes de brevet WO 96/15765, comme la 2,4,5,6-tétra-aminopyrimidine, la 4-hydroxy-2,5,6-triaminopyrimidine, la 2-hydroxy-4,5,6-triaminopyrimidine, la 2,4-dihydroxy-5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine; la 2,5-diméthyl-pyrazolo-[1,5-a]-pyrimidine-3,7-diamine ; la pyrazolo-[1,5-a]-pyrimidine-3,5-diamine ; la 2,7-diméthyl-pyrazolo-[1,5-a]-pyrimidine-3,5-diamine ; le 3-amino-pyrazolo-[1,5-a]-pyrimidin-7-ol ; le 3-amino-pyrazolo-[1,5-a]-pyrimidin-5-ol ; le 2-(3-amino pyrazolo-[1,5-a]-pyrimidin-7-ylamino)-éthanol; le 2-(7-amino-pyrazolo-[1,5-a]-pyrimidin-3-ylamino)-éthanol; le 2-[(3-amino-pyrazolo[1,5-a]pyrimidin-7-yl)-(2-hydroxy-éthyl)-amino]-éthanol; le 2-[(7-amino-pyrazolo[1,5-a]pyrimidin-3-yl)-(2-hydroxy-éthyl)-amino]-éthanol; la 5,6-diméthyl-pyrazolo-[1,5-a]-pyrimidine-3,7-diamine; la 2,6-diméthyl-pyrazolo-[1,5-a]-pyrimidine-3,7-diamine; la 2,5, N7, N7-tetraméthyl-pyrazolo-[1,5-a]-pyrimidine-3,7-diamine; la 3-amino-5-méthyl-7-imidazolylpropylamino-pyrazolo-[1,5-a]-pyrimidine; et leurs sels d'addition et leurs formes tautomères, lorsqu'il existe un équilibre tautomérique et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino-1-méthyl-pyrazole, le 3,4-diamino-pyrazole, le 4,5-diamino-1-(4'-chlorobenzyl)-pyrazole, le 4,5-diamino 1,3-diméthyl-pyrazole, le 4,5-diamino-3-méthyl-1-phényl-pyrazole, le 4,5-diamino 1-méthyl-3-phényl-pyrazole, le 4-amino-1,3-diméthyl-5-hydrazino-pyrazole, le 1-benzyl-4,5-diamino-3-méthyl-pyrazole, le 4,5-diamino-3-tert-butyl-1-méthyl-pyrazole, le 4,5-diamino-1-tert-butyl-3-méthyl-pyrazole, le 4,5-diamino-1-(β-hydroxyéthyl)-3-méthyl pyrazole, le 4,5-diamino-1-(β-hydroxyéthyl)-pyrazole, le 4,5-diamino-1-éthyl-3-méthyl-pyrazole, le 4,5-diamino-1-éthyl-3-(4'-méthoxyphényl)-pyrazole, le 4,5-diamino-1-éthyl-3-hydroxyméthyl-pyrazole, le 4,5-diamino-3-hydroxyméthyl-1-méthyl-pyrazole, le 4,5-diamino-3-hydroxyméthyl-1-isopropyl-pyrazole, le 4,5-diamino-3-méthyl-1-isopropyl-pyrazole, le 4-amino-5-(2'-aminoéthyl)amino-1,3-diméthyl-pyrazole, le 3,4,5-triamino-pyrazole, le 1-méthyl-3,4,5-triamino-pyrazole, le 3,5-diamino-1-méthyl-4-méthylamino-pyrazole, le 3,5-diamino-4-(β-hydroxyéthyl)amino-1-méthyl-pyrazole, et leurs sels d'addition avec un acide.

La composition selon l'invention comprend généralement une quantité totale de base(s) d'oxydation allant de 0,0005 à 12 % en poids par rapport au poids total de la composition. De préférence, elle comprend une quantité totale de base(s) d'oxydation allant de 0,005 à 8 % en poids, et mieux encore de 0,05 à 5 % en poids, par rapport au poids total de ladite composition.

Le ou les coupleurs utilisables dans la composition selon l'invention sont ceux classiquement utilisés dans les compositions de teinture d'oxydation, c'est-à-dire les méta-aminophénols, les méta-phénylènediamines, les métadiphénols, les naphtols et les coupleurs hétérocycliques tels que par exemple les dérivés indoliques, les dérivés indoliniques, le sésamol et ses dérivés, les dérivés pyridiniques, les dérivés pyrazolotriazoles, les pyrazolones, les indazoles, les benzimidazoles, les benzothiazoles, les benzoxazoles, les 1,3-benzodioxoles, les quinolines et les sels d'addition de ces composés avec un acide.

Ces coupleurs sont plus particulièrement choisis parmi le 2,4-diamino 1-(β-hydroxyéthyloxy)-benzène, le 2-méthyl-5-amino-phénol, le 5-N-(β-hydroxyéthyl)amino-2-méthyl-phénol, le 3-amino-phénol, le 1,3-dihydroxy-benzène, le 1,3-dihydroxy-2-méthyl-benzène, le 4-chloro-1,3-dihydroxy-benzène, le 2-amino 4-(β-hydroxyéthylamino)-1-méthoxy-benzène, le 1,3-diamino-benzène, le 1,3-bis-(2,4-diaminophénoxy)-propane, le sésamol, le 1-amino-2-méthoxy-4,5-méthylènedioxy benzène, l'α-naphtol, le 6-hydroxy-indole, le 4-hydroxy-indole, le 4-hydroxy-N-méthyl indole, la 6-hydroxy-indoline, la 2,6-dihydroxy-4-méthyl-pyridine, le 1-H-3-méthyl-pyrazole-5-one, le 1-phényl-3-méthyl-pyrazole-5-one, la 2-amino-3-hydroxypyridine, le 3,6-diméthyl-pyrazolo-[3,2-c]-1,2,4-triazole, le 2,6-diméthyl-pyrazolo-[1,5-b]-1,2,4-triazole et leurs sels d'addition avec un acide.

La composition selon l'invention comprend généralement une quantité totale de coupleur(s) allant de 0,0001 à 15 % en poids par rapport au poids total de la composition. De préférence, elle comprend une quantité totale de coupleur(s) allant de 0,001 à 10 % en poids, et mieux encore de 0,01 à 8 % en poids, par rapport au poids total de la composition.

D'une manière générale, les sels d'addition avec un acide des bases d'oxydation et des coupleurs sont notamment choisis parmi les chlorhydrates, les bromhydrates, les sulfates et les tartrates, les lactates et les acétates.

Selon un mode de réalisation particulier de l'invention, la composition comprend au moins une base d'oxydation et au moins un coupleur.

La composition tinctoriale conforme à l'invention peut en outre contenir un ou plusieurs colorants directs pouvant notamment être choisis parmi les colorants nitrés de la série benzénique, les colorants directs azoïques, les colorants directs méthiniques. Ces colorants directs peuvent être de nature non ionique, anionique ou cationique.

La composition conforme à la présente invention peut comprendre au moins un agent oxydant.

Un tel agent oxydant est choisi de préférence dans le groupe formé par le peroxyde d'hydrogène, le peroxyde d'urée, les bromates ou ferricyanures de métaux alcalins, les persels tels que les perborates et les persulfates.

L'utilisation du peroxyde d'hydrogène est particulièrement préférée. Cet agent oxydant est avantageusement constitué par une solution d'eau oxygénée dont le titre peut varier, plus particulièrement, d'environ 1 à 40 volumes, et encore plus préférentiellement d'environ 5 à 40.

On peut également utiliser à titre d'agent oxydant une ou plusieurs enzymes d'oxydoréduction telles que les laccases, les peroxydases et les oxydoréductases à 2 électrons (telles que l'uricase), éventuellement en présence de leur donneur ou cofacteur respectif.

Le milieu approprié pour la teinture appelé aussi support de teinture est un milieu cosmétique généralement constitué par de l'eau ou par un mélange d'eau et d'au moins un solvant organique acceptable sur le plan cosmétique. A titre d'exemples de solvants organiques, on peut notamment citer les alcools tels que l'alcool éthylique, l'alcool isopropylique, l'alcool benzylique, et l'alcool phényléthylique, ou les glycols ou éthers de glycol tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ainsi que les alkyléthers de diéthylèneglycol comme par exemple, le monoéthyléther ou le monobutyléther du diéthylèneglycol. Les solvants peuvent alors être présents dans des concentrations comprises entre environ 0,5 et 20 % et, de préférence, entre environ 2 et 10 % en poids par rapport au poids total de la composition.

La composition conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, autres que les polymères amphotères selon l'invention, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci-dessus sont en général présents en quantité comprise pour chacun d'eux entre 0,01 et 20 % en poids par rapport au poids de la composition tinctoriale.

Bien entendu, l'homme de l'art veillera à choisir le ou les éventuels composés complémentaires mentionnés ci-avant, de manière telle que les propriétés avantageuses attachées intrinsèquement aux compositions selon l'invention ne soient pas, ou substantiellement pas, altérées par la ou les adjonctions envisagées.

Le pH de la composition conforme à l'invention est généralement compris entre 3 et 12 environ, de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents alcalinisants, on peut citer, à titre d'exemple, l'ammoniaque, les carbonates alcalins, les alcanolamines telles que les mono-, di- et triéthanolamines ainsi que leurs dérivés, les hydroxyalkylamines et les éthylènediamines oxyéthylénées et/ou oxypropylénées, les hydroxydes de sodium ou de potassium et les composés de formule suivante : 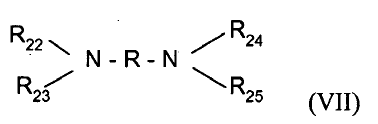 dans laquelle R est un reste propylène éventuellement substitué par un groupement hydroxyle ou un radical alkyle en C₁-C₄ ; R₂₂, R₂₃, R₂₄ et R₂₅, identiques ou différents, représentent un atome d'hydrogène, un radical alkyle en C₁-C₄ ou hydroxyalkyle en C₁-C₄.

Parmi les agents acidifiants, on peut citer, à titre d'exemple, des acides minéraux ou organiques comme l'acide chlorhydrique, l'acide orthophosphorique, des acides carboxyliques comme l'acide tartrique, l'acide citrique, l'acide lactique, ou des acides sulfoniques.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Le procédé de la présente invention est un procédé dans lequel on applique sur les fibres la composition selon la présente invention telle que définie précédemment, et on révèle la couleur à l'aide d'un agent oxydant. La couleur peut être révélée à pH acide, neutre ou alcalin et l'agent oxydant peut être ajouté à la composition de l'invention juste au moment de l'emploi ou il peut être mis en oeuvre à partir d'une composition oxydante le contenant, appliquée simultanément ou séquentiellement à la composition de l'invention. De préférence, cette coloration est révélée à pH neutre.

Selon un mode de réalisation particulier, la composition selon la présente invention est mélangée, de préférence au moment de l'emploi, à une composition contenant, dans un milieu approprié pour la teinture, au moins un agent oxydant, cet agent oxydant étant présent en une quantité suffisante pour développer une coloration. Le mélange obtenu est ensuite appliqué sur les fibres kératiniques. Après un temps de pose variant généralement de 1 à 60 minutes environ, de préférence 5 à 45 minutes environ, les fibres kératiniques sont rincées, lavées au shampooing, rincées à nouveau puis séchées.

L'invention a aussi pour objet un dispositif à plusieurs compartiments ou "kit" de teinture dans lequel un premier compartiment renferme la composition tinctoriale de la présente invention définie ci-dessus à l'exception de l'agent oxydant et un deuxième compartiment renferme une composition oxydante. Ce dispositif peut être équipé d'un moyen permettant de délivrer sur les cheveux le mélange souhaité, tel que les dispositifs décrits dans le brevet FR-2 586 913 au nom de la demanderesse.

La présente invention a également pour objet l'utilisation pour la teinture d'oxydation des fibres kératiniques, et en particulier des fibres kératiniques humaines telles que les cheveux, d'une composition tinctoriale telle que définie précédemment.

Les exemples qui suivent sont destinés à illustrer l'invention sans pour autant en limiter la portée.

Dans ces exemples, toutes les quantités sont indiquées en pour cent en poids de matière active (M.A.) par rapport au poids total de la composition, sauf indication contraire.

### Exemple 1 comparatif :

On a préparé deux compositions de teinture à partir des composés suivants (composition A comparative, composition B selon l'invention):

| **Composition** | **A** | **B** |
|---|---|---|
| alcool laurique oxyéthyléné à 12 OE commercialisé sous la dénomination REWOPAL 12 par la société GOLDSCHMIDT monostéarate de glycol | 7,5 | 7,5 |
| alcool oléocétylique oxyéthyléné à 30 OE commercialisé sous la dénomination EMULGIN O 30 par la société COGNIS | 3 | 3 |
| alcool décylique oxyéthyléné à 3 OE commercialisé sous la dénomination EMULGIN BL 309 par la société COGNIS | 10 | 10 |
| alcool cétylstéarylique (C16/C18 : 50/50) commercialisé sous la dénomination LANETTE O OR par la société COGNIS | 10 | 10 |
| acide laurique naturel | 2,5 | 2,5 |
| silice pyrogénée hydrophobe commercialisée sous la dénomination AEROSIL R972 par la société DEGUSSA | 1 | 1 |
| acide polyacrylique réticulé commercialisé sous la dénomination CARBOPOL 980 par la société NOVEON | 0,4 | 0,4 |
| propylène glycol | 10 | 10 |
| monoéthanolamine | 1,2 | 1,2 |
| polymère acrylamide / chlorure de diallyldiméthyl ammonium/acide acrylique (Merquat 3333 de Nalco) | - | 2,4 M.A. |
| acide diéthylène triamine pentacétique, sel pentasodique en solution aqueuse à 40% commercialisé sous la dénomination DISSOLVINE D40 par la société AKZO NOBEL | 2 | 2 |
| thiolactate d'ammonium en solution aqueuse à 58% (50% en acide thiolactique) | 0,8 | 0,8 |
| acide ascorbique | 0,2 | 0,2 |
| dioxyde de titane | 0,2 | 0,2 |
| ammoniaque à 20% de NH₃ | 10 | 10 |
| 2-méthyl-5-hydroxyéthyl aminophénol | 0,86 | 0,86 |
| p-aminophénol | 0,41 | 0,41 |
| 4-amino-2-hydroxytoluène | 0,57 | 0,57 |
| 6-hydroxyindole | 0,068 | 0,068 |
| p-phénylènediamine | 0,49 | 0,49 |
| résorcinol | 0,1 | 0,1 |
| parfum | qs | qs |
| eau | qsp 100 | qsp 100 |

On a mélangé dans un rapport pondéral 1+1,5 chacune des compositions A et B avec une composition oxydante titrant 20 volumes en eau oxygénée.

Les pH des mélanges ainsi obtenus sont de 10.

Ces mélanges sont appliqués sur des cheveux gris à 90 % de blancs pendant un temps de pose de 30 minutes à température ambiante.

Les cheveux sont ensuite rincés, lavés avec un shampooing standard, puis rincés à l'eau et séchés.

On obtient, avec les mélanges issus des compositions A et B avec la composition oxydante, une nuance à reflet cuivré rouge esthétique de bonne ténacité.

On observe que le mélange issu de la composition B avec la composition oxydante se rince plus facilement que celui obtenu avec la composition A.

En outre, après coloration avec le mélange issu de la composition B, on observe que les cheveux sont particulièrement lisses.

## Revendications

1. Composition pour la teinture d'oxydation des fibres kératiniques comprenant, dans un milieu approprié pour la teinture, au moins un colorant d'oxydation et au moins un polymère amphotère comprenant la répétition de :
(i) un ou plusieurs motifs issus d'un monomère de type acrylamide,
(ii) un ou plusieurs motifs issus d'un monomère de type halogénure de dialkyldiallylammonium, et
(iii) au moins 50 % en moles de un ou plusieurs motifs issus d'un monomère de type acide carboxylique vinylique.

2. Composition selon la revendication 1 dans laquelle les motifs issus d'un monomère de type acrylamide sont des motifs de structure (I) suivante: 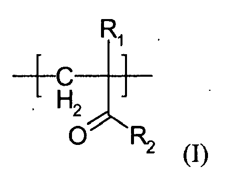 dans laquelle :
- R₁ désigne H ou CH₃,
- R₂ est choisi parmi un radical amino, diméthylamino, tert-butylamino, dodécylamino, ou
- NH-CH₂OH.

3. Composition selon la revendication 2 dans laquelle R₁ désigne H et R₂ est un radical amino.

4. Composition selon l'une quelconque des revendications précédentes dans laquelle les motifs issus d'un monomère de type halogénure de dialkyldiallylammonium sont des motifs de structure (II) suivante : 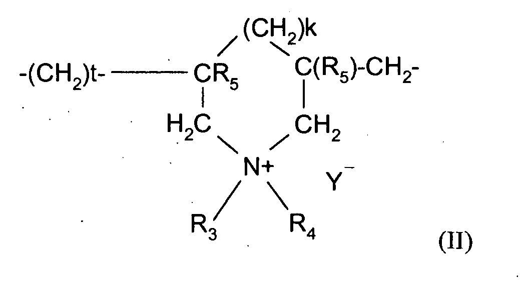 dans laquelle :
- k et t sont égaux à 0 ou 1, la somme k + t étant égale à 1 ;
- R₅ désigne un atome d'hydrogène ou un radical méthyle ;
- R₃ et R₄, indépendamment l'un de l'autre, désignent un groupement alkyle ayant de 1 à 4 atomes de carbone, un groupement hydroxyalkyle dans lequel le groupement alkyle a de 1 à 5 atomes de carbone, un groupement amidoalkyle(C₁-C₄), ou R₃ et R₄ forment conjointement avec l'atome d'azote auquel ils sont rattachés, des groupements hétérocycliques ;
- Y⁻ est un anion bromure, chlorure, acétate, borate, citrate, tartrate, bisulfate, bisulfite, sulfate ou phosphate.

5. Composition selon la revendication 4 dans laquelle les motifs issus d'un monomère de type halogénure de dialkyldiallylammonium sont des motifs dé formule (II) dans laquelle R₅ désigne un atome d'hydrogène et R₃ et R₄ désignent un radical méthyle, Y⁻ désignant un anion chlorure.

6. Composition selon l'une quelconque des revendications précédentes dans laquelle les motifs issus d'un monomère de type acide carboxylique vinylique sont choisis parmi les motifs de formule (III) : 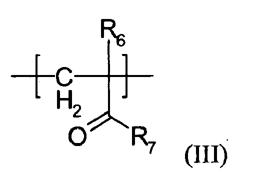 dans laquelle :
- R₆ désigne H ou CH₃,
- R₇ désigne un radical hydroxyle ou un radical -NH-C(CH₃)₂-CH₂-SO₃H.

7. Composition selon la revendication 6 dans laquelle le motif issu d'un monomère de type acide carboxylique vinylique est celui pour lequel R₆ désigne un atome d'hydrogène et R₇ désigne un radical hydroxyle.

8. Composition selon l'une quelconque des revendications précédentes dans laquelle le ou les polymères amphotères comprennent de 50 à 90 % en moles de motifs issus d'un monomère de type acide carboxylique vinylique.

9. Composition selon l'une quelconque des revendications précédentes dans laquelle le ou les polymères amphotères comprennent de 1 à 40 % en moles de motifs issus d'un monomère de type acrylamide.

10. Composition selon l'une quelconque des revendications précédentes dans laquelle le ou les polymères amphotères comprennent de 1 à 40 % en moles de motifs issus d'un monomère de type halogénure de dialkyldiallylammonium.

11. Composition selon l'une quelconque des revendications précédentes dans laquelle le ou les polymères amphotères sont choisis parmi les terpolymères acrylamide / chlorure de diméthyldiallylammonium / acide acrylique contenant au moins 50 % en moles de motifs issus de l'acide acrylique.

12. Composition selon l'une quelconque des revendications précédentes dans laquelle le ou les polymères amphotères sont présents en quantité comprise entre 0,1 et 10 % en poids par rapport au poids total de la composition de teinture.

13. Composition selon l'une quelconque des revendications précédentes dans laquelle le ou les colorants d'oxydation sont choisis parmi les bases d'oxydation et les coupleurs.

14. Composition selon la revendication 13 dans laquelle la ou les bases d'oxydation sont choisies parmi les ortho- et para-phénylènediamines, les bases doubles, les ortho- et para- aminophénols, les bases hétérocycliques ainsi que les sels d'addition de ces composés avec un acide.

15. Composition selon la revendication 13 ou 14 comprenant une quantité totale de base(s) d'oxydation allant de 0,0005 à 12 % en poids par rapport au poids total de la composition.

16. Composition selon l'une quelconque des revendications 13 à 15 dans laquelle le ou les coupleurs sont choisis parmi les méta-aminophénols, les méta-phénylènediamines; les métadiphénols, les naphtols, les coupleurs hétérocycliques et les sels d'addition de ces composés avec un acide.

17. Composition selon l'une quelconque des revendications 13 à 16 comprenant une quantité totale de coupleurs(s) allant de 0,0001 à 15 % en poids par rapport au poids total de la composition.

18. Composition selon l'une quelconque des revendications précédentes comprenant au moins un agent oxydant.

19. Composition selon la revendication 18 dans laquelle l'agent oxydant est choisi parmi le peroxyde d'hydrogène, le peroxyde d'urée, les bromates ou ferricyanures de métaux alcalins, les persels, les enzymes d'oxydoréduction avec éventuellement leur donneur ou cofacteur respectif.

20. Procédé de teinture des fibres kératiniques, **caractérisé en ce qu'**une composition telle que définie à l'une quelconque des revendications 1 à 17 est appliquée sur les fibres kératiniques en présence d'un agent oxydant pendant un temps suffisant pour développer la coloration désirée.

21. Dispositif à plusieurs compartiments dans lequel un premier compartiment contient une composition telle que définie à l'une quelconque des revendications 1 à 17 et un deuxième compartiment contient un agent oxydant.

22. Utilisation pour la teinture d'oxydation des fibres kératiniques d'une composition telle que définie à l'une quelconque des revendications 1 à 19.

## Claims

1. Composition for the oxidation dyeing of keratinous fibres comprising, in a medium appropriate for dyeing, at least one oxidation dye and at least one amphoteric polymer comprising the repetition of:
(i) one or more units resulting from a monomer of acrylamide type,
(ii) one or more units resulting from a monomer of dialkyldiallylammonium halide type, and
(iii) at least 50 mol% of one or more units resulting from a monomer of vinylcarboxylic acid type.

2. Composition according to Claim 1, in which the units resulting from a monomer of acrylamide type are units with the following structure (I): 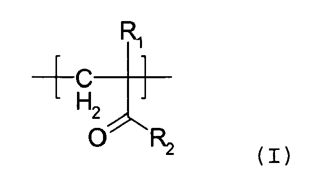 in which :
- R₁ denotes H or CH₃,
- R₂ is chosen from an amino, dimethylamino, tert-butylamino, dodecylamino or -NH-CH₂OH radical.

3. Composition according to Claim 2, in which R₁ denotes H and R₂ is an amino radical.

4. Composition according to any one of the preceding claims, in which the units resulting from a monomer of dialkyldiallylammonium halide type are units with the following structure (II): 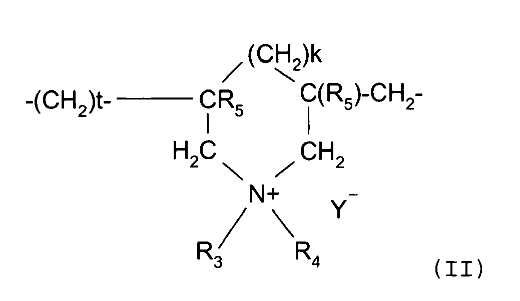 in which:
- k and t are equal to 0 or 1, the sum k + t being equal to 1;
- R₅ denotes a hydrogen atom or a methyl radical;
- R₃ and R₄ denote, independently of one another, an alkyl group having from 1 to 4 carbon atoms, a hydroxyalkyl group in which the alkyl group has from 1 to 5 carbon atoms, or an amido(C₁-C₄)alkyl group, or R₃ and R₄ form, jointly with the nitrogen atom to which they are attached, heterocyclic groups;
- Y⁻ is a bromide, chloride, acetate, borate, citrate, tartrate, bisulphate, bisulphite, sulphate or phosphate anion.

5. Composition according to Claim 4, in which the units resulting from the monomer of dialkyldiallylammonium halide type are units of the formula (II) in which R₅ denotes a hydrogen atom and R₃ and R₄ denote a methyl radical, Y⁻ denoting a chloride anion.

6. Composition according to any one of the preceding claims, in which the units resulting from a monomer of vinylcarboxylic acid type are chosen from the units of formula (III) : 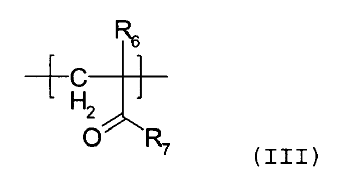 in which:
- R₆ denotes H or CH₃,
- R₇ denotes a hydroxyl radical or an -NH-C(CH₃)₂-CH₂-SO₃H radical.

7. Composition according to Claim 6, in which the unit resulting from a monomer of vinylcarboxylic acid type is that for which R₆ denotes a hydrogen atom and R₇ denotes a hydroxyl radical.

8. Composition according to any one of the preceding claims, in which the amphoteric polymer or polymers comprise from 50 to 90 mol% of units resulting from a monomer of vinylcarboxylic acid type.

9. Composition according to any one of the preceding claims, in which the amphoteric polymer or polymers comprise from 1 to 40 mol% of units resulting from a monomer of acrylamide type.

10. Composition according to any one of the preceding claims, in which the amphoteric polymer or polymers comprise from 1 to 40 mol% of units resulting from a monomer of dialkyldiallylammonium halide type.

11. Composition according to any one of the preceding claims, in which the amphoteric polymer or polymers are chosen from acrylamide/dimethyldiallylammonium chloride/acrylic acid terpolymers comprising at least 50 mol% of units resulting from acrylic acid.

12. Composition according to any one of the preceding claims, in which the amphoteric polymer or polymers are present in an amount of between 0.1 and 10% by weight, with respect to the total weight of the dyeing composition.

13. Composition according to any one of the preceding claims, in which the oxidation dye or dyes are chosen from oxidation bases and couplers.

14. Composition according to Claim 13, in which the oxidation base or bases are chosen from ortho- and para-phenylenediamines, double bases, ortho- and para-aminophenols, heterocyclic bases and the addition salts of these compounds with an acid.

15. Composition according to Claim 13 or 14, comprising a total amount of oxidation base(s) ranging from 0.0005 to 12% by weight, with respect to the total weight of the composition.

16. Composition according to any one of Claims 13 to 15, in which the coupler or couplers are chosen from meta-aminophenols, meta-phenylenediamines, meta-diphenols, naphthols, heterocyclic couplers and the addition salts of these compounds with an acid.

17. Composition according to any one of Claims 13 to 16, comprising a total amount of coupler(s) ranging from 0.0001 to 15% by weight, with respect to the total weight of the composition.

18. Composition according to any one of the preceding claims, comprising at least one oxidizing agent.

19. Composition according to Claim 18, in which the oxidizing agent is chosen from hydrogen peroxide, urea hydrogen peroxide, alkali metal bromates, alkali metal ferricyanides, persalts, and oxidation-reduction enzymes with optionally their respective donor or cofactor.

20. Method for dyeing keratinous fibres, **characterized in that** a composition as defined in any one of Claims 1 to 17 is applied to keratinous fibres in the presence of an oxidizing agent for a time sufficient to develop the desired colouring.

21. Multicompartment device, in which a first compartment comprises a composition as defined in any one of Claims 1 to 17 and a second compartment comprises an oxidizing agent.

22. Use for the oxidation dyeing of keratinous fibres of a composition as defined in any one of Claims 1 to 19.

## Patentansprüche

1. Zusammensetzung zum oxidativen Färben von Keratinfasern, die in einem zum Färben geeigneten Medium mindestens einen Oxidationsfarbstoff und mindestens ein amphoteres Polymer enthält, das als Wiederholungseinheiten aufweist:
(i) eine oder mehrere Einheiten, die von einem Monomer vom Typ Acrylamid stammen,
(ii) eine oder mehrere Einheiten, die von einem Monomer vom Top Dialkyldiallylammoniumhalogenid stammen, und
(iii) mindestens 50 Mol-% einer oder mehrerer Einheiten, die von einem Monomer vom Typ Vinylcarbonsäure stammen.

2. Zusammensetzung nach Anspruch 1, wobei die Einheiten, die von einem Monomer vom Typ Acrylamid stammen, Einheiten der folgenden Struktur (I) sind: 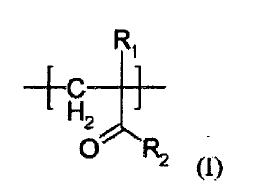 in der Formel:
R₁ bedeutet H oder CH₃,
R₂ ist unter den Gruppen Amino, Dimethylamino, *tert*-Butylamino, Dodecylamino oder -NH-CH₂OH ausgewählt.

3. Zusammensetzung nach Anspruch 2, wobei R₁ H bedeutet und R₂ eine Aminogruppe ist.

4. Zusammensetzung nach einem der vorhergehenden Ansprüche, wobei die Einheiten, die von einem Monomer vom Typ Dialkyldiallylammoniumhalogenid stammen, Einheiten der folgenden Struktur (II) sind: 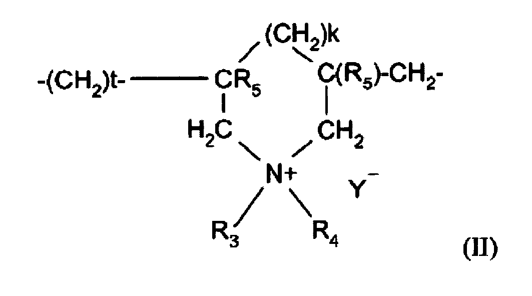 in der Formel:
· k und t sind 0 oder 1, wobei die Summe k + t 1 bedeutet;
· R₅ bedeutet ein Wasserstoffatom oder die Methylgruppe;
· R₃ und R₄ bedeuten unabhängig voneinander eine Alkylgruppe mit 1 bis 4 Kohlenstoffatomen, eine Hydroxyalkylgruppe, bei der die Alkylgruppe 1 bis 5 Kohlenstoffatome aufweist, eine Amidoalkyl(C₁₋₄)gruppe, oder R₃ und R₄ bilden gemeinsam mit dem Stickstoffatom, an das sie gebunden sind, heterocyclische Gruppen;
· Y⁻ bedeutet Bromid, Chlorid, Acetat, Borat, Citrat, Tartrat, Bisulfat, Bisulfit, Sulfat oder Phosphat.

5. Zusammensetzung nach Anspruch 4, wobei die Einheiten, die von einem Monomer vom Typ Dialkyldiallylammoniumhalogenid stammen, Einheiten der Formel (II) sind, worin R₅ ein Wasserstoffatom bedeutet und R₃ und R₄ Methyl bedeuten, wobei Y-Chlorid ist.

6. Zusammensetzung nach einem der vorhergehenden Ansprüche, wobei die Einheiten, die von einem Monomer vom Typ Vinylcarbonsäure stammen, unter den Einheiten der Formel (III) ausgewählt sind: 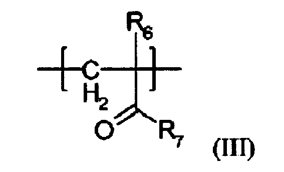 in der Formel:
R₆ bedeutet H oder CH₃,
R₇ bedeutet die Hydroxygruppe oder eine Gruppe -NH-C(CH₃)₂-CH₂-SO₃H.

7. Zusammensetzung nach Anspruch 6, bei der die Einheit, die von einem Monomer vom Typ Vinylcarbonsäure abgeleitet ist, eine Einheit ist, bei der R₆ ein Wasserstoffatom bedeutet und R₇ die Hydroxygruppe ist.

8. Zusammensetzung nach einem der vorhergehenden Ansprüche, wobei das oder die amphotere(n) Polymer(e) 50 bis 90 Mol-% Einheiten enthalten, die von einem Monomer vom Typ Vinylcarbonsäure stammen.

9. Zusammensetzung nach einem der vorhergehenden Ansprüche, wobei das oder die amphotere(n) Polymer(e) 1 bis 40 Mol-% Einheiten enthalten, die von einem Monomer vom Typ Acrylamid stammen.

10. Zusammensetzung nach einem der vorhergehenden Ansprüche, wobei das oder die amphotere(n) Polymer(e) 1 bis 40 Mol-% Einheiten enthalten, die von einem Monomer vom Typ Dialkyldiallylammoniumhalogenid stammen.

11. Zusammensetzung nach einem der vorhergehenden Ansprüche, wobei das oder die amphotere(n) Polymer(e) unter den Acrylamid/Dimethyldiallylammoniumchlorid/Acrylsäure-Terpolymeren, die mindestens 50 Mol-% von Acrylsäure stammende Einheiten enthalten, ausgewählt sind.

12. Zusammensetzung nach einem der vorhergehenden Ansprüche, wobei das oder die amphotere(n) Polymer(e) in einem Mengenanteil im Bereich von 0,1 bis 10 Gew.-%, bezogen auf das Gesamtgewicht der Farbmittelzusammensetzung, enthalten sind.

13. Zusammensetzung nach einem der vorhergehenden Ansprüche, **dadurch gekennzeichnet, dass** der oder die Oxidationsfarbstof(e) unter den Oxidationsbasen und Kupplern ausgewählt sind.

14. Zusammensetzung nach Anspruch 13, bei der die Oxidationsbase(n) unter den ortho- und para-Phenylendiaminen, Doppelbasen, ortho- und para-Aminophenolen, heterocyclischen Basen und den Additionssalzen dieser Verbindungen mit einer Säure ausgewählt sind.

15. Zusammensetzung nach Anspruch 13 oder 14 mit einer Gesamtmenge an Oxidationsbase(n) im Bereich von 0,0005 bis 12 Gew.-%, bezogen auf das Gesamtgewicht der Zusammensetzung.

16. Zusammensetzung nach einem der Ansprüche 13 bis 15, wobei der oder die Kuppler(n) unter den meta-Aminophenolen, meta-Phenylendiaminen, meta-Dihydroxybenzolen, Naphtholen, heterocyclischen Kupplern und den Additionssalzen dieser Verbindungen mit einer Säure ausgewählt sind.

17. Zusammensetzung nach einem der Ansprüche 13 bis 16 mit einer Gesamtmenge an Kuppler(n) im Bereich von 0,0001 bis 15 Gew.-%, bezogen auf das Gesamtgewicht der Zusammensetzung.

18. Zusammensetzung nach einem der vorhergehenden Ansprüche, die mindestens ein Oxidationsmittel enthält.

19. Zusammensetzung nach Anspruch 18, wobei das Oxidationsmittel unter Wasserstoffperoxid, Harnstoffperoxid, Alkalimetallbromaten, Alkalimetallferricyaniden, Salzen von Persäuren und Redoxenzymen gegebenenfalls in Gegenwart ihres jeweiligen Donors oder Cofaktors ausgewählt ist.

20. Verfahren zum Färben von Keratinfasern, **dadurch gekennzeichnet, dass** eine Zusammensetzung, wie sie in einem der Ansprüche 1 bis 17 definiert ist, während einer Zeitspanne, die ausreichend ist, um die gewünschte Färbung zu bilden, in Gegenwart eines Oxidationsmittels auf die Keratinfasern aufgebracht wird.

21. Vorrichtung mit mehreren Abteilungen, bei der eine Abteilung eine Zusammensetzung enthält, wie sie einem der Ansprüche 1 bis 17 definiert ist, und eine zweite Abteilung ein Oxidationsmittel enthält.

22. Verwendung einer Zusammensetzung, wie sie einem der Ansprüche 1 bis 19 definiert ist, zum oxidativen Färben von Keratinfasern.
